# EUROPEAN PATENT APPLICATION

(11) **EP 1 403 222 A1**
(43) Date of publication of application: **31.03.2004**
(21) Application number: 02745825.6
(22) Date of filing: 04.07.2002
(51) Int. Cl.: C02F 11/04, B01D 3/00

(54) **METHOD OF ANAEROBICALLY TREATING ORGANIC MATERIAL AND ANAEROBIC TREATMENT APPARATUS**

(30) Priority: 05.07.2001 JP 2001204921
(71) Applicant: NKK CORPORATION, Tokyo 100-0005 (JP)
(72) Inventor: FUJIWARA, Shigeki, c/o NKK Corporation, Chiyoda-ku, Tokyo 100-0005 (JP); TSUJI, Takeshi, c/o NKK Corporation, Chiyoda-ku, Tokyo 100-0005 (JP); TSUBONE, Toshiaki, c/o NKK Corporation, Chiyoda-ku, Tokyo 100-0005 (JP)
(74) Representative: HOFFMANN - EITLE
(86) International application number: PCT/JP2002/006786
(87) International publication number: WO 2003/004423

(57) **Abstract**

In order to realize the treatment for an organic material anaerobically, which contains a big amount of a solid matter, and in order to deal with the organic material at a high decomposition rate and at a lower cost, the method and apparatus provides: the steps of: solubilizing the solid organic material in the organic material by alkali treatment to obtain the soluble organic material; applying acid fermentation to the organic material containing the soluble organic material, obtained in the solubilization step, by anaerobic bacteria; separating the material obtained in the acid fermentation step into the solid component and into the liquid component; applying methane fermentation to the liquid component obtained in the separation step for obtaining the digestive gas; introducing the digestive gas to the acid fermentation step, introducing the generating ammonia in the acid fermentation step into the digestive gas; and taking out the ammonia, together with the digestive gas from the acid fermentation system, thus removing the ammonia; and recycling the solid component obtained in the separation step to the solubilization step.

## Description

### FIELD OF THE INVENTION

The present invention relates to one technology regarding an anaerobic digestion of organic materials, which includes sewage sludge, dewatered cake of sewage sludge, night soil, night soil sludge, septic tank sludge, and garbage.

### BACKGROUND OF THE INVENTION

Conventionally, reaction tanks for digesting anaerobically various sorts of materials such as sewage sludge, dewatered cake of sewage sludge, night soil, night soil sludge, septic tank sludge, and garbage, have been applied to a single-phase complete mixing (single-stage) method, a single-phase plug flow method, a two-phase method, and the like.

The anaerobic digestion technology makes it possible to recover energy (to recover methane gas) from the treated material. And the anaerobic digestion technology provides prospective and effective views of reducing the generated quantity of a solid substance and stabilizing the generated solid substance itself , thus the technology has been noticeable as one of the important technologies in the resource-recycle-oriented society, today. However, the anaerobic digestion technology, has some problem-issues, which are, a low reaction rate in the anaerobic digestion, a need for a large-sized facility, and a necessity of energy for heating the facility.

With regard to the anaerobic digestion technology, increasing the concentration of solid matter in the reaction tank for methane fermentation invites a significant effect on downsizing the reaction facility. Simultaneously, it invites a significant effect no reducing the energy consumption amount for heating.

On the other hand, increasing the concentration ratio of the solid matter in the reaction tank raises the following problem. That is, at the same time, the concentration of ammonia increases in the reaction tank, which reduces the efficiency of the anaerobic digestion, particularly which reduces the volume of a generated gas.

Up to now, the most serious problem of the anaerobic digestion technology has been how to deal with inhibition by ammonia, which has a bad influence on the efficiency by the anaerobic digestion. Owing to the inhibition of ammonia, a material, which contains a large amount of ammoniac components, is difficult to be treated for methane fermentation. And a material, which contains a large amount of ingredients that generate ammonia, (such as proteins), is also difficult to be treated for methane fermentation.

With respect to the above-mentioned problems, there has been disclosed such a technology as increasing the concentration of solid matter in the reaction tank and as removing ammonia in the reaction tank to suppress the inhibition of ammonia. This is disclosed in Japanese Patent Laid-Open No. 7-185585. However, looking the disclosed invention into detail, removing the ammonia is conducted by dewatering operation after being treated under the high temperature, which increases the cost to remove the ammonia. Consequently, the useful effects to be obtained cannot be expected , from the judging-point of the expenditures to remove the ammonia (The anaerobic digestion, in case of a high concentration ratio the solid matter , is available for, to some extent, tough.)

Another sort of technology is known to the world. That is, to increase the activity of microorganisms having the ability to generate methane by adding iron salt, cobalt salt, nickel salt, or the like to the treated material, in order to suppress generating the ammonia, which is an inhibitor against the methane fermentation. However, the technology also needs a great deal of cost for spending on chemicals. The chemicals are such as iron salt, cobalt salt, and nickel salt. Therefore, there is very few advantage. Supplementary speaking, the technology cannot be effective and prosperous, by the reason that there exists the limited amount of the solid matter concentration ratio, which is, around 9%, no matter how the higher concentration ratio is aimed at. That's to say, it cannot be expected to get a significant increase in the concentration ratio of the solid matter.

Consequently, it has not been realized to increase the concentration of solid matter in the reaction tank for methane fermentation, on the practical stage to be used.

Furthermore, when the objective organic materials are the sewage sludge, the dewatered cake of the sewage sludge, the septic tank sludge, or the like, the size of the organic material as the treated material is, from time to time, not small enough for the microorganisms to live in the acid fermentation and in the methane fermentation. As the result, it is difficult to decompose completely the organic materials, which exist in those kinds of treated materials.

### SUMMARY OF THE INVENTION

An object of the present invention is to solve the above-described problems and to provide a method for treating an organic material at lower cost and in a high efficiency for realizing the treatment method and the apparatus for dealing with the organic material, which has a high concentration ratio of the solid matter and a high decomposition rate of the organic material.

The present invention provides a treatment method and an apparatus thereof, as follows.
(1) A method for treating an organic material anaerobically comprising the steps of: obtaining a soluble organic material by treating a solid organic material in an organic material by an alkali-treatment; applying an acid fermentation by an anaerobic bacteria, to the obtained soluble organic material treated in the obtaining step; applying a methane fermentation to the organic material treated in the acid fermentation step, to obtain a digestive gas; introducing the digestive gas into the acid fermentation step; removing an ammonia, by introducing the ammonia produced in the acid fermentation step into the digestive gas introduced into the acid fermentation step, and by taking out the ammonia together with the digestive gas from the acid fermentation step.
(2) The method for anaerobically treating organic material according to (1), further has the solid-liquid separation step between the acid fermentation step and the methane fermentation step. The solid-liquid separation step has means for separating the organic material treated in the acid fermentation step into a solid component and into a liquid component, and for transporting the liquid component into a tank of the methane fermentation step.
(3) The method for anaerobically treating organic material according to (2), further has the step of recycling thus separated solid component to the step of obtaining the soluble organic material.
(4) The method for anaerobically treating organic material according to (1), further has the step of passing the digestive gas containing ammonia, taken out from the acid fermentation system during the ammonia removing step, through an alkali to absorb carbon dioxide and a volatile organic material in the digestive gas.
(5) The method for anaerobically treating organic material according to (4), further has the step of passing the digestive gas containing the ammonia, after passing through the step of absorbing the carbon dioxide and the volatile organic material, through an acid to absorb the ammonia.
(6) The method for anaerobically treating organic material according to (4) or (5), further has the step of using the alkali, used for absorbing the carbon dioxide and the volatile organic material, as an alkali for alkali treatment in the solubilization step.
(7) The method for anaerobically treating organic material according to (1), wherein the alkali treatment is conducted within a range of reaction pH from 8 or more to 12 or less.
(8) The method for anaerobically treating organic material according to (1), wherein the alkali treatment is conducted using sodium hydroxide.
(9) An apparatus for anaerobically treating an organic material by anaerobic treatment contains: a solubilization tank to apply alkali treatment to a solid organic material in the treating material to obtain a soluble organic material; an acid fermentation tank to apply acid fermentation to the organic material containing the soluble organic material by anaerobic bacteria; a methane fermentation tank to apply methane fermentation to the material obtained in the acid fermentation tank to obtain a digestive gas; and an ammonia-removing means for introducing the digestive gas obtained in the methane fermentation step to the acid fermentation tank, for taking ammonia produced in the acid fermentation tank into the digestive gas, and for taking out the ammonia together with the digestive gas from the acid fermentation tank.
(10) The apparatus for anaerobically treating organic material according to (9), further has a solid-liquid separation device, inserted in the passage connecting the acid fermentation tank with the methane fermentation tank, for separating the material obtained in the acid fermentation tank into a solid component and a liquid component; the solid-liquid separation device has a passage connecting to the acid fermentation tank to receive the organic material after treated by acid fermentation, and a passage connecting to the methane fermentation tank to transfer the liquid component thereto.
(11) The apparatus for anaerobically treating organic material according to (9) further has a passage for recycling the solid component obtained in the solid-liquid separation device to the solubilization tank.
(12) The apparatus for anaerobically treating organic material according to (9), further has a carbon dioxide and volatile organic material absorption tank to absorb the carbon dioxide and the volatile organic material in the digestive gas by passing the digestive gas containing the ammonia, taken out from the acid fermentation tank by ammonia-removing means, through an alkali.
(13) The apparatus for anaerobically treating organic material according to (9), further has an ammonia absorption tank to absorb the ammonia in the digestive gas by passing the digestive gas, from which the carbon dioxide and the volatile organic material were removed in the carbon dioxide and volatile organic material absorption tank, through an acid.
(14) The apparatus for anaerobically treating organic material according to (12) or (13), further has a passage for transferring the alkali, which absorbed the carbon dioxide and the volatile organic material in the digestive gas, from the carbon dioxide and volatile organic material absorption tank to the solubilization tank.

### BRIEF DESCRIPTION OF THE DRAWINGS

FIG. 1 is a schematic drawing of an example, related to the configuration of the anaerobic treatment apparatus, according to the present invention.
FIG. 2 is a schematic drawing of another example, related to the configuration of the anaerobic treatment, according to the present invention.

### DESCRIPTION OF THE PREFERRABLE EMBODIMENT

Referring to each drawing, the present invention is described as follows, in detail. The organic material being treated according to the present invention is an organic material, which contains a solid organic material, or the organic material such as sewage sludge, dewatered cake of sewage sludge, night soil, night soil sludge, septic tank sludge, garbage, and food waste. The treatment method according to the present invention has the solubilization step for applying alkali treatment to the solid organic material in the organic material, in order to obtain a soluble organic material. By way of solubilizing the solid organic material in the treated organic material, the hydrolysis and acid-generation in the acid fermentation step (described below) are enhanced.

In the solubilization step, a heat treatment and/or an ultrasonic treatment can be used, simultaneously with alkali treatment. By applying the alkali treatment, while applying the heat treatment and/or the ultrasonic treatment, in some cases, it can be obtained to shorten the reaction time and to increase the solubilized percentage. The reaction time, the reaction temperature, and the reaction pH are arbitrarily determined, depending on the decomposed percentage of the organic material to be aimed at. However, the reaction pH in the solubilization tank is, preferably within a range from 8 or more to 12 or less, and the reaction temperature during the heating treatment is preferably from 50ºC or more to 85ºC or less. The alkali, which is used in the alkali treatment, is not especially specified, but sodium hydroxide is preferable, from the standing viewpoint of treating and maintaining easily.

The treated method, according to the present invention, has the acid fermentation step, following the solubilization step. The acid fermentation step is the step for generating an organic acid through the acid fermentation of the organic material as the treated material, under the presence of anaerobic microorganisms. The methanogenic bacteria convert the organic material into an organic acid. The methanogenic bacteria easily decompose the organic acid. According to the present invention, the solid organic material in the organic material is solubilized in the solubilization step, as described above. So, the hydrolysis and the acid-generation in the acid fermentation step are enhanced. As the result, the anaerobic digestion improves efficiently.

The organic material, which has passed through the acid fermentation step, is introduced into the methane fermentation step.

In the methane fermentation step, the organic acid is anaerobically decomposed in the methane fermentation tank by the action of methanogenic bacteria, and is converted to the digestive gas, which contains methane and carbon dioxide. The retention time and the temperature in the methane fermentation tank are not especially specified the retention time and the temperature are, optionally, determined, judging from the properties and characteristics of the introduced organic material.

The treating method according to the present invention has the step for removing ammonia. In the step for removing ammonia the digestive gas generated in the methane fermentation step is introduced into the methane fermentation step. And the ammonia is taken into the digestive gas. (Here, the above-mentioned ammonia generates by the process of the hydrolysis and the acid generation, in the acid fermentation step.) As described above, the ammonia is harmful to the microorganisms, which carry out the hydrolysis and the acid generation in the acid fermentation step. As mentioned above, it is, already, known as the most serious problem that the higher concentration ratio of the ammonia inhibits the anaerobic digestion efficiently. However, in the present invention, the step for removing the ammonia suppresses increasing the concentration of the ammonia in the acid fermentation tank. And, it makes it possible to treat the organic material anaerobically, under the condition of the high concentration of the solid matter and by the lower investment-cost and running-cost.

In the acid fermentation step, not only the alkali but the organic material, which contains volatile fatty acids such as acetic acid and propionic acid, (hereinafter defined as the "volatile organic material") generates. When the ammonia is taken into the digestive gas in the step for removing the alkali, the volatile organic material, is, also, taken into the digestive gas to be taken out from the acid fermentation system.

Preferably, the treatment disclosed in the present invention has more kind of the step, that is, the step for absorbing the carbon dioxide and the volatile organic material in the digestive gas. The step is achieved by way of passing the digestive gas taken out from the acid fermentation system in the step for removing ammonia through an alkali. The alkali, which absorbed the carbon dioxide and the volatile organic material, is preferably re-used as the alkali for the alkali treatment in the solubilization step. All the volatile organic material is decomposed and is used for the methanogenic bacteria, the organic material, whose molecular weight decreases in the acid, can be utilized in the methane fermentation, without wasting the amount.

Additionally speaking, in the treatment method of the present invention, it is preferable to pass the digestive gas containing the ammonia, which has already passed trough the step for absorbing the carbon dioxide and the volatile organic material, through the acid. And then, it is preferable to have the step for absorbing the ammonia, which has generated in the acid fermentation step. The method leads to a good result to use the ammonia effectively. However, such a step for absorbing the ammonia does not always need the previous step for absorbing the carbon dioxide and the volatile organic material.

With respect to the above-mentioned steps, even if the sequence of the both step is reversible, and even if merely the step for absorbing the ammonia is achieved, it can be attained to make use of the ammonia efficiently.

Supplementary speaking, in the treatment process of the present invention, it is preferable to have the step for adding the gas that does not contain an oxygen, (for example, nitrogen, carbon dioxide, and hydrogen), onto the digestive gas being introduced into the acid fermentation step.

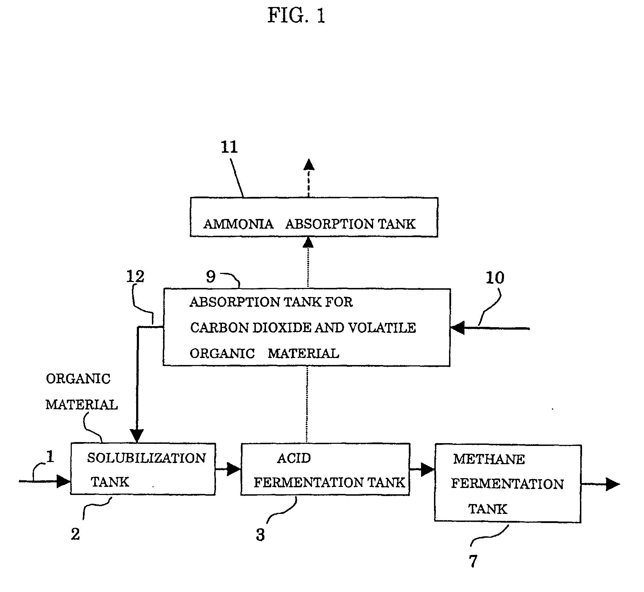

In accordance with the Drawings, the present invention is explained in detail, as follows. FIG.1 is a schematic drawing of an example of configuration of the anaerobic treatment apparatus, according to the present invention. The organic material 1 as the treated material is supplied into the solubilization tank 2, where the alkali treatment is applied to the organic material 1 in order to solubilize the solid organic material in the organic material. The organic material, which contains the obtained soluble organic material in this way, is introduced into the acid fermentation tank 3. The alkali treatment, which is conducted in the solubilization tank, can be achieved, together with the heat treatment and/or the ultrasonic treatment, depending on the object of the necessity. In such a step for solubilizing, the hydrolysis and the acid generation enhance in the acid fermentation tank 3.

In the acid fermentation tank 3, the organic material containing the soluble organic material is treated anaerobially. In this way, an easy-decomposing organic material can be obtained by the action of the methanogenic bacteria in the solubilization tank 2. The methanogenic bacteria in the methane fermentation tank 7 anaerobically decompose the organic acid, in order to get the digestive gas that contains the methane and the carbon dioxide. The figure of the methane fermentation tank 7 is not especially specified. The figure of the methane fermentation tank 7 can be chosen, depending on the characteristics of the introduced organic material. However, from the viewpoint of reacting, the preferable types of the methane fermentation tank 7 include an upward-flow anaerobic sludge blanket (UASB) reactor and an expanded granular sludge bed (EGSB) reactor.

The digestive gas 8, which generates in the methane fermentation tank 7, is introduced into the acid fermentation tank 3. And the digestive gas discharges therefrom, as a state of a mixture of the digestive gas 8 and the ammonia, which generates in the acid fermentation tank 3, thus taking out the generating ammonia from the acid fermentation system.

Owing to the step for removing ammonia, treating anaerobically the organic material with high concentration ratio of the solid matter becomes available at a lower cost. The volatile organic materials such as the acetic acid and the propionic acid (as well as the alkali) generate, inside of the acid fermentation tank 3. Accordingly, the volatile material is also taken into the digestive gas, and is discharged outside of the acid fermentation tank 3.

The treatment facility shown in FIG. 1 has the carbon dioxide and the absorption tank for volatilizing the organic material 9, the transfer passage for transporting alkali 12 (which is the alkali 10 after having absorbed the carbon dioxide and the volatile organic material), and the ammonia absorption tank 11. The above-mentioned elements are optional, according to the present invention, though.) The digestive gas taken out from the acid fermentation tank 3 is transported into the absorption tank for absorbing the carbon dioxide and the volatile organic material 9, where the alkali 10 absorbs the carbon dioxide and the volatile organic material in the digestive gas. The alkali, which has been used in the absorption tank for absorbing the carbon dioxide and the volatile organic material 9 is transported into the solubilization tank 2, via the passage for transporting alkali 12, and is used as the alkali for the alkali treatment. In the system, the organic material, whose molecular weight has decreased in the acid fermentation step, can be recycled and re-used as one sort of the gas for the methane fermentation without wasteful materials.

The digestive gas, from which the carbon dioxide and the volatile organic material are removed, leads to the ammonia absorption tank 11, where the digestive gas absorbs the ammonia. After that, the digestive gas may be introduced into a gasholder (not shown in the FIG.). The digestive gas can be often used as a fuel for an electric-power generator or a boiler. The ammonia, which exists in the ammonia absorption tank 11, is absorbed, by way of using the acid. The kind of the acid is not specified in the definite one. Following the example, a sulfuric acid is used, from time to time. The digestive gas stored in the gas-holder can be recycled and re-used into the acid fermentation tank 3, as the gas for agitation or as the gas for removing the ammonia, when the demand comes on.

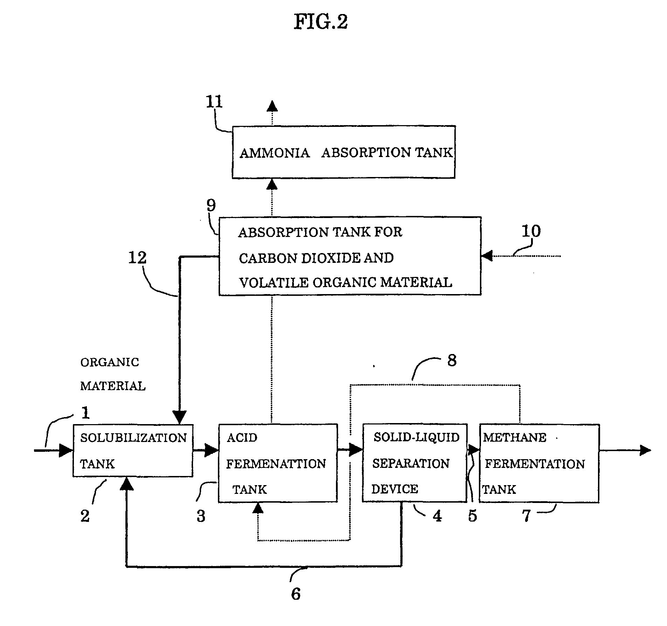

The facility shown in Fig. 2 is one example of adopting the solid-liquid separation device, according to the present invention. (The reference symbols other than those for the solid-liquid separation device and relating ones are the same with those in Fig. 1, and the numeric corresponding description is not written up here.) The solid-liquid separation device 4 separates the fermented liquid obtained in the acid fermentation tank 3, splitting into a liquid component and a solid component. The separation device 4 may be a centrifugal separator, a filter cloth separator, a membrane separator, and a gravitational concentrator. However, the type of the separation device 4 is not specified herein.

The step for separating the solid and the liquid separates the fermented liquid into two sorts, which means, one is the separated solid component, the other is the separated liquid component. The separated liquid component is introduced into the methane fermentation step, and the separated solid component is introduced into the solubilization step to be recycled. Introducing the liquid component into the methane fermentation step improves the processing rate of the methane fermentation. Contrarily, introducing the solid component into the solubilization step to be recycled can bring up the high percentage of the decomposed organic material, which has been introduced into the solubilization step.

The methanogenic bacteria in the methane fermentation tank 7, anaerobically decompose the liquid component that has been separated into the separation device 4, in order to generate the digestive gas that contains the methane and the carbon dioxide. On the other hand, the solid component, which has been separated into the separation device 4, is returned back to the solubilization tank 2, via the solid component recycle passage 6, in order to be re-used in the solubilization treatment.

### [Examples]

In more detail, the present invention is written up, referring to the following examples.

At first, a wastewater treatment facility, which has the system configuration in the present invention, is shown in Fig. 2. On the contrary, as the comparative example against the present invention, it was done to use a normal methane fermentation method, so called, by single-tank & medium-temperature (35°C), that is well known to the world as a common technology. (Hereinafter, the comparative example is defined as "the comparative system"). In the example of the present invention, the organic material used in the experiment was a dewatered cake of sewage sludge, which had the characteristic of 10 mass% as the content of the solid matter. In the comparative example of the comparative system, the dewatered cake was prepared and adjusted to the degree of the characteristic, which means, 5 mass% as the content of the solid matter. This adjustment was taken into consideration, by the reason that the ammonia did not inhibit the comparative system.

The sample sludge was treated by in the acid fermentation tank, by the alkali- treatment. The treatment was done, under the condition that the temperature was 80ºC and that the duration of the alkali-treatment was for 30 minutes. The result was as follows;
The effect by the solubilization reached 70%, in comparison with the index value, which was [soluble COD_{Cr}=/Total COD_{Cr}]. (Note: COD_{Cr} is defined as a COD value, which is measured by using a potassium di-chromate as an oxidizing reagent.)

The prepared soluble sludge was introduced into the acid fermentation tank , where the acid fermentation tank had already been heated to the degree of 55°C, and where SRT(Solid Retention Time) was continued to be for 3 days in the acid fermentation tank. In the situation, the acid fermentation had been done. Until the time when a satisfactory digestive gas was produced in the UASB reactor (as the device followed by the acid fermentation tank), a nitrogen gas was injected into the acid fermentation tank, at an injection-rate of 100 Liter/Liter-Reactor per one hour. In this way, stripping the generated ammonia was carried out.

After the acid fermented the sludge, the sludge was separated into the liquid component and into the solid component in the centrifugal separator. The liquid component was introduced into the UASB reactor, whose effective capacity was 6 Liter, and whose heated value was to the degree of 35ºC, in order to go for the methane fermentation. The UASB reactor was operated, under the conditions that the volume load was 10 kg-COD_{Cr}/m³/day and that HRT (Hydraulic Retention Time) was for 6 days.

With regard to the comparative system, the experiment was carried out, while using a 20-Liter reactor at 35ºC for 20 days of SRT (Solid Retention Time).

The experimental results are shown in Table 1.

**Table 1**

| | Unit | Example | Comparative Example |
|---|---|---|---|
| Total retention time | day | 9 | 20 |
| Introduced TS concentration ratio | mass% | 10 | 5 |
| Introduced VS concentration ratio | mass% | 8.1 | 4 |
| Generation amount of Methane gas | L/kg Introduced VS | 510 | 300 |
| Note) TS: Total solid matter. VS: Volatile solid matter, that's to say, organic solid matter. | | | |

As shown in Table 1, it can be found out clearly that the methane amount, which generates per the corresponding introduced organic material, has 1.8 times that of the related prior art. Not only the increasing and generating methane amount, but the concentration ratio of the introduced solid matter can be brought up to be higher. In addition, the present invention makes it possible to shorten the reaction days for treating the organic materials.

### INDUSTRIAL APPLICABILITY

As described above, the present invention assures treating method for the organic material, with high concentration of the solid matter. And the present invention provides a high decomposition percentage of the organic material, thus the present invention provides a method and an apparatus for treating the organic material to achieve the down-sizing of the facility. Furthermore, the heating cost can be economized and the recovery rate for the digestive gas increases. Additionally, the method and the apparatus of the present invention reduce the volume of the organic material in an extreme efficient manner, which was usually dealt with by incineration and/or landfill, up to now. So, the landfill area can be kept for a long time, by the reason of reducing the volume of the organic material. And the energy consumption amount for incinerating the organic materials can be economized. Thus, the method and the apparatus of the present invention also invite an excellent effect on the waste treatment technology. Consequently, the present invention is very significant, from the standing viewpoint of the industrial value.

## Claims

1. A method for treating an organic material anaerobically comprising the steps of:
obtaining a soluble organic material by treating a solid organic material in an organic material by an alkali-treatment;
applying an acid fermentation by an anaerobic bacteria, to the obtained soluble organic material treated in the obtaining step;
applying a methane fermentation to the organic material treated in the acid fermentation step, to obtain a digestive gas;
introducing the digestive gas into the acid fermentation step;
removing an ammonia, by introducing the ammonia produced in the acid fermentation step into the digestive gas introduced into the acid fermentation step, and by taking out the ammonia together with the digestive gas from the acid fermentation step.

2. The method according to claim 1, further comprising the step of separating the organic material treated in the acid fermentation step into a solid component and into a liquid component, between the acid fermentation step and the methane fermentation step, to transport the liquid component into a methane fermentation tank.

3. The method according to claim 2 further comprising the step of recycling the separated solid component to the step of obtaining the soluble organic material.

4. The method according to claim 1, further comprising the step of passing the digestive gas containing the ammonia, which is taken out from the acid fermentation step, through an alkali to absorb a carbon dioxide and a volatile organic material in the digestive gas.

5. The method according to claim 4, further comprising the step of passing the digestive gas containing the ammonia through an acid to absorb the ammonia, after passing the digestive gas containing the ammonia through the step of absorbing the carbon dioxide and the volatile organic material.

6. The method according to claim 4 or 5 further comprising the step of transporting the alkali used for absorbing the carbon dioxide and the volatile organic material, into the step of obtaining the soluble organic material, for re-using the alkali by the alkali treatment in the step of obtaining the soluble organic material.

7. The method according to claim 1, wherein pH value in the alkali treatment is within a range from 8 or more to 12 or less.

8. The method according to claim 1, wherein the alkali treatment uses a sodium hydroxide.

9. An apparatus for treating an organic material anaerobically comprising:
a solubilization tank for treating a solid organic material in the organic material by an alkali treatment, to obtain a soluble organic material;
an acid fermentation tank, connected with the solubilization tank by a passage, for applying the acid fermentation to the organic material containing the soluble organic material, by anaerobic bacteria;
a methane fermentation tank, connected with the acid fermentation tank for conducting the methane fermentation by using the organic material obtained in the acid fermentation tank, for obtaining a digestive gas;
a passage for introducing the digestive gas obtained in the methane fermentation tank into the acid fermentation tank; and,
means for removing an ammonia by taking the ammonia produced in the acid fermentation tank into the digestive gas obtained in the methane fermentation tank and by taking out the ammonia together with the digestive gas from the acid fermentation tank.

10. The apparatus according to claim 10 further comprising a solid-liquid separation device, located between the acid fermentation tank and the methane fermentation tank, connected to the acid fermentation tank for separating the material obtained in the acid fermentation tank into a solid component and into a liquid component, and connected to the methane fermentation tank via a passage for transporting the liquid component.

11. The apparatus according to claim 9 further comprising a passage for returning back the solid component obtained in the solid-liquid separation device into the solubilization tank.

12. The apparatus according to claim 9 further comprising an absorption tank for absorbing the carbon dioxide and the volatile organic material in the digestive gas taken out from the acid fermentation tank, by passing the digestive gas containing the ammonia, through an alkali by the means for removing the ammonia.

13. The apparatus according to claim 9 further comprising an ammonia absorption tank for absorbing the ammonia in the digestive gas removing the carbon dioxide and the volatile organic material, by passing through an acid.

14. The apparatus according to claim 12 or 13 further comprising a passage for transporting the alkali absorbing the carbon dioxide and the volatile organic material in the digestive gas, from the absorption tank for absorbing the carbon dioxide and volatile organic material into the solubilization tank.
